(19) Europäisches Patentamt
European Patent Office
Office européen des brevets

(11) Publication number: **0 099 742**
**A1**

(12) # EUROPEAN PATENT APPLICATION

(21) Application number: 83304135.3

(22) Date of filing: 15.07.83

(51) Int. Cl.[3]: C 12 P 19/36
C 12 P 1/00

(30) Priority: 17.07.82 GB 8220760

(43) Date of publication of application:
01.02.84 Bulletin 84/5

(84) Designated Contracting States:
AT BE CH DE FR GB IT LI LU NL SE

(71) Applicant: GRAND METROPOLITAN BIOTECHNOLOGY LTD.
430 Victoria Road
South Ruislip Middlesex(GB)

(72) Inventor: Knowles, Christopher John
42 Somner Close
Canterbury Kent(GB)

(74) Representative: Baillie, Iain Cameron et al,
c/o Ladas & Parry Isartorplatz 5
D-8000 München 2(DE)

(54) **Enzymatic reaction process.**

(57) A biochemical enzymatic reaction involving an oxidizing enzyme which converts a substrate to an oxidized product with $NAD^+$ or $NADP^+$ as coenzyme is provided with a recycling system for returning the NADH or NADPH produced to the oxidized state by the presence of a NADH or NADPH oxidase. The use of the oxidase to recycle the coenzyme avoids the presence of undesirable additional substrates and by-products and in general the oxidation potential of the reverse reaction enhances the basic enzymatic oxidation.

EP 0 099 742 A1

Croydon Printing Company Ltd.

ENZYMATIC REACTION PROCESS

This invention relates to enzymatic reactions.

A large number of enzymes require an oxidised or reduced nicotinamide adenine dinucleotide ($NAD^+$ or NADH) or nicotinamide adenine dinucleotide phosphate ($NADP^+$ or NADPH) as a cofactor or coenzyme. Thus in the biochemical processes in which these enzymes are used to convert substrates to oxidized products, stoichiometric amounts of $NAD^+$ or $NADP^+$ must be employed. The necessity for supplying these amounts of expensive cofactors is a significant factor in the economic and industrial use of these enzymes for oxidation reactions.

To some extent, the problem can be overcome by introducing a second enzyme and a cosubstrate to recycle the cofactor. For example, alcohol dehydrogenase and malate dehydrogenase can be used (O. Campbell and T.M.S. Chang, Biochemical and Biophysical Research Communications 69 (1976) 562) as shown in the flow diagram in Fig. 1. In such a system only catalytic amounts of $NAD^+$ are required providing they are retained in the bioreaction system. However, the desired product from the reaction e.g. acetaldehyde has to be separated from the reduction product of the reverse reaction e.g. malate in the above reaction, which requires costly down-stream processing; moreover cosubstrate in oxidized form (oxaloacetate in the above example) must be supplied in excess to drive the reaction to completion which increases the cost apart from being disadvantageous in regard to separation of products.

The problem of coproduct formation can be overcome by employing as the reverse reaction one which will result in NADH formation but a non-interfering by-product. For example one can use NADH peroxidase with peroxide as a cosubstrate:

$$NADH + H^+ + H_2O_2 \longrightarrow NAD^+ + 2H_2O$$

In this system there is no need to separate the desired reaction products from the product stream since the product is water. On the other hand it still is necessary to provide a cosubstrate which in this instance is peroxide. Moreover, peroxides are toxic to many enzyme reactions and inactivate free and immobilized enzymes which may reduce dramatically the concentration of the substrates which can be used and the lifetime of the enzyme reaction, again increasing the potential cost of the system.

It has now been found that NADH oxidase which catalyses the reaction:

$$2NADH + 2H^+ + O_2 \longrightarrow 2NAD^+ + 2H_2O$$

can be used to recycle NADH produced in such a system. Similarly NADPH can be recycled by NADPH oxidase.

According to the invention therefore there is provided an enzymatic process for the production of an oxidised product by reaction of an oxidising enzyme on a corresponding substrate in the presence of $NAD^+$ or $NADP^+$ wherein the reduced cofactor is returned to the oxidised state by a soluble flavoprotein enzyme which is NADH oxidase or NADPH oxidase in the presence of a source of oxygen.

The system is diagramatically illustrated in Fig. 2 for the $NAD^+$ system.

Significant cost improvements are achieved since no cosubstrate is required other than oxygen or an oxygen source which is usually normally present in the system and co-substrates and co-products are not required so avoiding the necessity of any separation of product from by-product or co-product. Moreover the kinetics of the system are such that the oxidation potential of the return or recycle reaction is usually greater than that of the enzymatic reaction using the oxidising enzyme so as to urge that system towards completion.

The system of the invention can be applied to a wide variety of reactions involving oxidizing enzymes.

The relevant main or basic enzymes of the system are the oxido-reductases of Class 1 of the Recommendation as to Enzyme Nomenclature of the Nomenclature Committee of the International Union of Biochemistry (the IUB system) sometimes known as dehydrogenases which enzymes require $NAD^+$ or $NADP^+$ as a cofactor in the transition reaction.

For example the system can be applied to the production of acetaldeyhyde from alcohol or alpha-ketoglutarate and ammonia from glutamate employing alcohol dehydrogenase and glutamate dehydrogenase respectively. Other oxidizing enzymes or dehydrogenases could be glycerol phosphate dehydrogenase, lactate dehydrogenase, glyceraldehyde-3-phosphate dehydrogenase, glutamate dehydrogenase ($NADP^+$), glycerol dehydrogenase, glucose dehydrogenase, glucose dehydrogenase ($NADP^+$), beta-hydroxy steroid dehydrogenase, oestradiol -17-beta dehydrogenase, aryl alcohol ($NADP^+$) dehydrogenase, aldehyde dehydrogenase, lactaldehyde dehydrogenase, arylaldehyde dehydrogenase, cortisone-beta-reductase, cholestenone-5 beta-reductase, 1-aminoacid dehydrogenase but these are merely exemplary. The main enzyme and the recyle enzyme (e.g. NADH oxidase) should naturally be reasonably compatable to appropriate conditions for effective reaction, e.g. temperature and pH and there should not be any enzymatic interference between the systems but such can be readily determined from the existing knowledge on enzymes or by simple testing.

The relative proportions of the NADH oxidase or the NADPH oxidase to the basic oxidising enzyme and the NAD or $NAD^+$ present should be such that the basic oxidising action is pushed to completion as far as possible. Under most enzymatic process conditions the difference in redox potential of $NADH/NAD^+$ or $NADPH/NADP^+$ couples and the $H_2O/O_2$ couple is so large that the basic oxiding enzymatic reaction will be driven to completion by the action of the NADH oxidase or NADPH oxidase.

Naturally, as indicated above, the conditions in the biochemical enzymatic system must be such to favour both enzymatic reactions as far as practicable. It is therefore desirable, that as far as possible the NADH oxidase or NADPH oxidase operates in optimum conditions of pH temperature and concentration of components as does the oxidizing enzyme reaction with which it is cooperating. The precise choice of operating conditions however will offer no problem to one skilled in the art given the extensive knowledge on the optimum condition for each oxidising enzyme.

As preferred conditions the system can be operated at a pH of from 6 to 9 and temperature $15^{o}$ to $45^{o}C$ particularly 6.5 to 8.0 pH, 20 to $30^{o}C$ but the available variation is only limited by the practicalities of enzymatic reactions and the optimisation of desired yields and efficiencies.

The reaction can be carried out in a wide variety of conventional bioreactors. Because of the expense of replacement for the $NAD^+$ or $NADP^+$ the construction of the bioreaction system will be such as to minimize any loss of cofactor from the system, for example by use of membrane-filter systems or hollow fibre systems. The cofactor can also be stabilized in this system by linking to either of the enzymes or a base material so as to provide effectively either free or 'stabilized' products.

Thus the cofactor or coenzyme can be stabilized in this system by chemical linking by additional chains so as to enlarge the molecular size of the cofactor so that it will not pass easily through a semi-permeable membrane or by linking into the enzyme or onto an independent base on which the enzyme is immobilized or otherwise linked using lengthy side chains where appropriate.

Typical reaction systems in which the invention in the process canbe carried out can be columns or stirred tanks or other types of bioreactors. The enzymatic system according to the invention can also be employed in other systems utilizing enzymatic reactions. For example a sensing device for presence of materials or activity of enzymes can be devised by applying a combination of oxidising enzymes, cofactor and NAPH or NAPH to a sensor or electrode sensitive to a change produced by the activity of the system.

Thus, where an appropriate substrate is present for the oxidising enzyme, the consequent production of NADH, for example, and recycle to $NAD^+$ with the utilization of oxygen could be measured by, for example an oxygen electrode, so providing means of measuring the concentration of the substrate in the system.

The source of oxygen for the recycle of the NADH or NADPH i.e. for reaction involving the NAPH oxidase or NADPH oxidase can be oxygen added to the system from air or oxygenated gas or can be derived,at least in part, from an oxygen carrier providing this carrier does not other-wise interfere with the enzymatic system.

The NADH oxidase or NADPH oxidase can be secured in a known manner. NADH oxidase has been derived from lactic acid bacteria (S.Y.R.Pugh and C.J.Knowles, Journal of General Microbiology (1982) 1009; D.D. Hoskins, H.R. Whiteley and D. Mackler, Journal Biological Chemistry 237 (1962) 2647. Thus the enzyme can be derived from lactic acid bacteria of the Family Streptocoocaeae, genus Streptococcus, genus Leuconostoc, and genus Pedicoccus, and of the Family Lactobacillaceae, genus Lactobacillus in the sense of Bergey's Manual of Determinative Bacteriology 8th edition. Many strains of bacteria produce or contain a membrane bound complex of NADH oxidase or NADPH oxidase as part of the respiratory system of the cell. These species of lactic acid bacteria form a soluble flavoprotein NADH oxidase enzyme. Leuconostoc mesenteroides, National Collection of Industrial and Marine Bacteria (NClMB) strain 8699 contains particularly high levels of the enzyme and can be used routinely. Also Streptococcus faecalis NClMB 8661 can be used; one skilled in the art is well aware of the appropriate strains which would give enzymes of the greatest activity.

For the production of the enzyme bacteria may be grown in many common media and preferably can be grown aerobically but also anaerobically. Bacteria can then be harvested in exponential or stationary phase and resuspended in a buffer. If desired the enzyme can be recovered from the bacteria which are broken down by common procedures such as sonication, osmotic lysis with lysozyme, grinding with sand or in a pressure cell. The crude-cell free extract can be used or a supernatant fraction from the high-speed centrifugation at 150,000g for 90 minutes, or a pure NADH oxidase could be prepared by standard purification procedures.

Activity of a NADH oxidase from Leuconostoc mesenteroides strain NClMB 8699 procured in the above manner would be active between pH 6 and 9 and from 15° to 45°C, preferably 6.5 to 8.0 pH and 20° to 30°C. Specific activities in cell-free extracts, supernatant fraction and the purified protein are 1.0 to 4.0, 2.0 to 7.0 and 50 to 100 μmol NADH oxidised per minute mg protein, respectively.

The invention will now be illustrated in the following Examples:

EXAMPLE 1

Recycling in an Alcohol Dehydrogenase System.

In this system alcohol i.e. ethanol is oxidised to acetaldehyde while the NADH oxidase converts NADH to $NAD^+$ with oxygen from the system. NADH oxidase was prepared from Leuconostoc mesenteroides strain NClMB 8699. The bacteria were grown aerobically at 30°C in a medium containing 2% glucose, 1.5% yeast extract, 1.5% nutrient broth, 0.2% $K_2HPO_4$, 0.2% triammonium citrate, 0.02% $MgSO_4.7H_20$ and 0.02% $MnSO_4.4H_20$. The bacteria were broken by a sonication procedure and a cell free extract prepared. To a mixture of from 20°C to 30°C of $NAD^+$(1 μmol per ml), ethanol (1 to 100 μmol per ml), buffer (100 μmol per ml,) having a pH of from 7.5 to 8.5 and alcohol dehydrogenase (3.5 units per ml, where one unit converts 1 μmole substrate (i.e. ethanol) to product in 1 minute) are added 100 μl of the above cell-free extract. After one hour 70% or more of the alcohol was converted to acetaldehyde and after 3 hours essentially all the alcohol is converted to acetaldehyde. The resulting acetaldehyde was free of any major amounts of undesirable by-product.

EXAMPLE 2

The process of Example 1 was repeated using a supernatant fraction NADH oxidase extract. Substantially similar results were obtained.

EXAMPLE 3

The procedure in Example 1 was repeated with a semi-purified NADH oxidase and substantially similar results were obtained.

EXAMPLE 4

The procedure in Example 1 was repeated using an NADH oxidase derived from Streptococcus faecalis NClMB 8661 using a cell-free extract; substantially similar results were obtained.

EXAMPLE 5

Procedure for the production of alpha-ketoglutarate and ammonia from glutamate.

To a mixture at 20°C to 30°C of $NAD^+$ (0.3 µmol per ml), glutamate (1 to 50 umol per ml), buffer (100 µmol , pH 7.5 to 8.5) and glutamate dehydrogenase (7.2 units per ml) were added 100 µl cell-free extract of NADH oxidase derived from Leuconostoc mesenteroides as described in Example 1. After 30 minutes the yield of alpha-ketoglutarate was 50% of the glutamate.

This was repeated with supernatant fraction and purified NADH oxidase with substantially similar results.

CLAIMS:

1. A process for preparing an oxidised product from a substrate employing an oxidising enzyme with a cofactor which is $NAD^+$ or $NADP^+$ in which the resulting NADH or NADPH is returned to the oxidised state by the enzymatic reaction of a soluble flavoprotein enzyme which is NADH oxidase or NADPH oxidase in the presence of a source of oxygen.

2. A process according to claim 1 in which the NADH oxidase is derived from lactic acid bacteria.

3. A process according to claim 2 in which the bacteria belong to the genera Leuconostoc or Streptococcus.

4. A process according to any one of the preceding claims in which the oxidation reaction is conversion of ethanol to acetaldehyde by alcohol dehydrogenase.

5. A process according to any one of claims 1 to 3 wherein the enzyme reaction is the conversion of glutamate to alpha-ketoglutarate by glutamate dehydrogenase.

6. A process according to any one of claims 1 to 5 conducted at a pH of 6 to 9 and at a temperature of 15 to 45°C.

7. A process according to any one of the preceding claims in which the oxidising enzyme and the NADH or $NADPH_2$ oxidases are stabilised by immobilisation.

8. A process according to any one of the preceding claims in which the $NAD^+$ or $NADP^+$ is contained in a bioreactor by a semipermeable membrane.

9. A process according to any one of claims 1 to 7 wherein the $NAD^+$ or $NADP^+$ is maintained in the reactor by enlarging the molecular size of the cofactor.

10. A process according to any one of claims 1 to 7 in which the cofactor molecule is anchored to an inert base or to an enzymatic component by means of a molecular extension.

11. A process according to any one of claims 1 to 10 in which the reaction system is mounted on a sensor or electrode sensitive to a change produced by the reaction system to provide a sensing device.

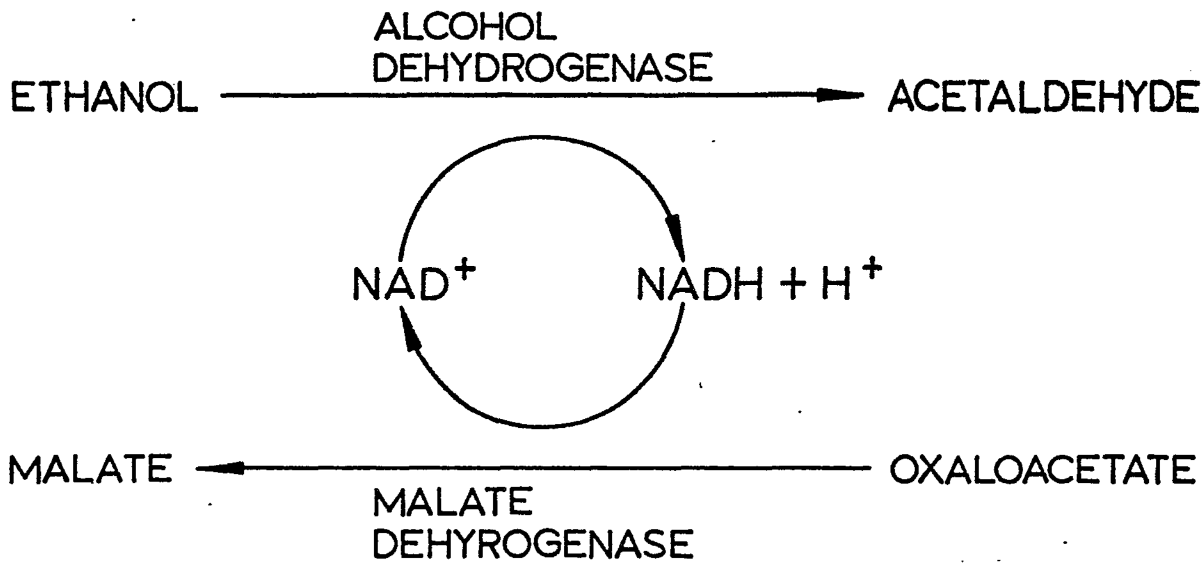
ALCOHOL
DEHYDROGENASE
ETHANOL
ACETALDEHYDE
NAD+
NADH + H+
MALATE
OXALOACETATE
MALATE
DEHYROGENASE

Fig.1

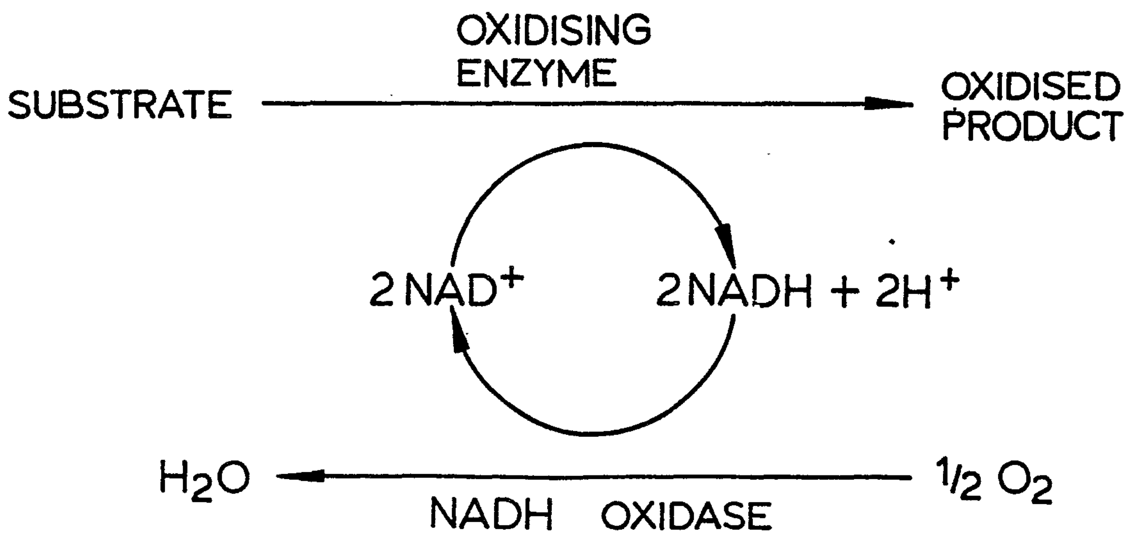
OXIDISING
ENZYME
SUBSTRATE
OXIDISED
PRODUCT
2 NAD+
2 NADH + 2H+
H2O
1/2 O2
NADH OXIDASE

Fig.2

0099742

European Patent Office

# EUROPEAN SEARCH REPORT

Application number

EP 83 30 4135

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | FR-A-2 472 607 (ANVAR)<br>* Page 8, claims 1-5; example 1; page 2, line 13 - page 4, line 37 *<br>--- | 1,4,6<br>7,8 | C 12 P 19/36<br>C 12 P 1/00 |
| X | CHEMICAL ABSTRACTS, vol. 83, no. 17, 27 October 1975, page 200, no. 143688e, Columbus, Ohio, USA<br>K. KAWAI et al.: "Preparation of immobilized NADH oxidase and its use as a regenerator" & HAKKO KOGAKU ZASSHI 1975, 53(8), 588-94<br>* Abstract *<br>--- | 1-4,7<br>10 | |
| X | ANALYTICAL LETTERS, vol. 9, (3), 1976, pages 257-276, Marcel Dekker, Inc.<br>W.H. BARICOS et al.: "Practical processing with cofactor-requiring enzymes" * Page 260, paragraph 1; page 261, last paragraph;and especially page 262, second half; pages 267-269, the whole content *<br>--- -/- | 1-4,6-<br>10 | TECHNICAL FIELDS SEARCHED (Int. Cl. 3)<br>C 12 P<br>C 12 D<br>C 12 B |

The present search report has been drawn up for all claims

| Place of search | Date of completion of the search | Examiner |
|---|---|---|
| THE HAGUE | 18-10-1983 | DEKEIREL M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

0099742

European Patent Office

# EUROPEAN SEARCH REPORT

Application number

EP 83 30 4135

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | CHEMICAL ABSTRACTS, vol. 97, no. 11, September 1982, pages 614,615, no. 90392j, Columbus, Ohio, USA S.H. GWAK et al.: "Immobilization of an NADH-oxidizing complex and its use as a regenerator of NAD" & J. FERMENT. TECHNOL. 1982, 60(3), 205-10 * Abstract * --- | 1,4,7, 10 | |
| A | FR-A-2 217 295 (INSTITUT FRANCAIS DU PETROLE, DES CARBURANTS ET LUBRIFIANTS) * Page 8, claims 1,6; page 1, lines 25-30; page 2, line 31 - page 3, line 1 * ----- | 1 | |
| | | | TECHNICAL FIELDS SEARCHED (Int. Cl. 3) |

The present search report has been drawn up for all claims

| Place of search | Date of completion of the search | Examiner |
|---|---|---|
| THE HAGUE | 18-10-1983 | DEKEIREL M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document